# EUROPEAN PATENT SPECIFICATION

(11) **EP 1 531 758 B1**
(45) Date of publication and mention of the grant of the patent: **17.02.2010**
(21) Application number: 03771680.0
(22) Date of filing: 21.07.2003
(51) Int. Cl.: A61F 2/00

(54) **TISSUE REPAIR DEVICE WITH A REMOVABLE SUPPORT MEMBER**
GEWEBEIMPLANTAT MIT ABZIEHBARER TRÄGERFOLIE
DISPOSITIF DE REPARATION DE TISSUS A ELEMENT DE SUPPORT AMOVIBLE

(30) Priority: 25.07.2002 US 206523
(43) Date of publication of application: 25.05.2005
(73) Proprietor: Gore Enterprise Holdings, Inc., Newark, DE 19714-9206 (US)
(72) Inventor: FARNSWORTH, Ted, Ray, Flagstaff, AZ 86001 (US); HUPPENTHAL, Joseph, A., Flagstaff, AZ 86004 (US); WALTER, James, T., Flagstaff, AZ 86001 (US)
(74) Representative: Shanks, Andrew
(86) International application number: PCT/US2003/022744
(87) International publication number: WO 2004/010896

(56) References cited:
- WO-A-02/078568
- DE-U- 9 012 161
- US-A- 5 433 996
- US-A- 5 501 661

## Description

### FIELD OF THE INVENTION

The present invention relates to implantable medical devices. In particular, the invention is directed to a composite implantable medical device having an implantable tissue repair component and a component that assists in deployment of the tissue repair component at a surgical site and is readily removable from the tissue repair component following deployment.

### BACKGROUND OF THE INVENTION

Implantable medical devices designed to help repair damaged tissues have been successfully used for years. Some of these devices are used to patch damaged tissue and provide mechanical support to the tissues during healing. A simple form of these devices is a flexible patch-like sheet. The composition and morphology of the sheets are usually tailored to address a particular surgical need. In some cases, it is desirable to incorporate a reinforcing element into the flexible sheets. In addition to mechanical support, reinforcing elements often assist in handling and deployment of the implantable sheet devices. Ease of handling and deployment of flexible sheet devices are particularly desired when laproscopic instruments and procedures are used to implant the devices.

An example of one of these devices is disclosed by de la Torre in U.S. Patent No. 5,368,602. de la Torre describes a patch made of a surgical mesh material having one or more semi-rigid frame-like support elements permanently secured to the mesh material along all or part of the border of the material. The semi-rigid support members are said to enable manipulation and positioning of the entire mesh area with conventional laproscopic instruments.

U.S. Patent No. 5,695,525, issued to Mulhauser et al., discloses a semi-rigid framework in the form of a ring permanently attached to one side of a planar mesh material. The support ring is designed to prevent the planar mesh material from collapsing into crater-like defects in tissue by maintaining the mesh material in an expanded configuration across the defect site.

A repair patch similar to the Mulhauser et al. device is disclosed in U.S. Patent No. 5,824,082, issued to Brown. The Brown patch utilizes a framework made of a metallic support wire. The support wire has shape-memory properties. The support wire is permanently attached to a preformed patch material along its periphery. The shape-memory characteristic of the support wire enables the repair patch to be rolled into a small cylindrical profile at room temperatures and alter its configuration to expand and flatten the patch material at body temperatures. The repair patch is said to reside between layers of tissue at a repair site and not require sutures or staples.

In U.S. Patent No. 6,280,453, issued to Kugel et al., a hernia repair patch is disclosed having the form of a laminated mesh material with a framework made of a resilient monofilament spring permanently located between layers of the laminate. Once the patch is placed through an incision site in a hernia patient, the spring element assists in unfolding and expanding the patch into a planar configuration. The patch is provided with a pouch into which a surgeon can place a finger to position the patch across a hernia, rather than having to use a laproscopic instrument to position the patch.

A variation on the theme of implantable tissue repair devices having permanently attached reinforcing frameworks is disclosed by Gianturco in U.S. Patent No. 5,258,000.
The Gianturco device is initially implanted as an unsupported flexible bag having an internal space into which an elastic stiffener wire is subsequently threaded. The stiffener wire causes the bag to adopt a flattened shape. The flattened repair device with its stiffener wire is permanently secured to tissue surrounding the repair site with sutures or staples.

In surgical procedures that utilize a tissue repair material with a stiffener element, it may be desirable to remove the stiffener element from the repair material following at least partial attachment of the repair material to tissues of the repair site. In U.S. Patent No. 5,370,650, issued to Tovey et al., an apparatus for positioning tissue repair meshes adjacent to body tissue is disclosed. The apparatus includes a delivery device with an arm that extends to place the tissue repair mesh into a surgical site. The arm has a stiffener element for the tissue repair mesh attached to its distal end. The tissue repair mesh is secured to the stiffener element with sutures sewn around the stiffener element and through holes in the mesh. The sutures can be sewn in such a way as to permit removal of the sutures from the tissue repair mesh following deployment. Prior to removal of the sutures from the mesh material, at least a portion the mesh material can be secured to tissues of the surgical site with sutures or staples. Once the tissue repair mesh is in place, the sutures holding the mesh to the stiffener element are removed. The delivery device is then separated from the tissue repair mesh and extracted from the surgical site.

A pneumatically operated deployment device for a tissue repair mesh is disclosed in U.S. Patent No. 6,302,897, issued to Rousseau. The Rousseau device is an applicator with a tissue repair mesh simply placed on an external surface of an inflatable bladder. The bladder has two portions. The first portion is filled with air. The second portion is initially empty, but is fillable with the air from the first portion when external mechanical pressure is applied to the first portion. As the second portion is inflated, the applicator and tissue repair mesh are unfolded and the repair mesh pressed against a patient's tissue. Following deployment of the mesh, the bladder is removed from the surgical site by hand.

Another mesh is disclosed in US-A-5 433 996.

None of these devices recognize the advantages of providing an implantable sheet material with a resilient support member that is releasably adhered to the sheet material. Such a device would have an adhesion scheme that permits the resilient support member to be initially held in place on the implantable sheet material with sufficient strength to withstand placement within and delivery from a laproscopic or similar surgical instrument. Yet the adhesion scheme would have sufficient weakness to permit the support member to be removed from the implantable sheet material with conventional surgical techniques following deployment of the device at a surgical site.

The resilient support member would assist in changing the implantable sheet material from a compacted configuration to a more planar configuration. The device would also provide an unobstructed border area in which a complete set of sutures or staples encompassing the repair material could be put in place and tested before the support element is removed from the repair material. Such a device would optionally include features that assist in tactile and visual orientation of the device at a surgical site.

WO-A-02078568 is state of the art according to Article 54(3) EPC and describes a surgical implant suitable for treatment of hernias. The implant comprises a mesh which may be releasably attached to a backing strip which may be formed from plastics material. Following the placement of the mesh the backing strip can be removed from the mesh, the mesh being retained on the body and the backing material being removed by the surgeon.

### SUMMARY OF THE INVENTION

The present invention is directed to a medical device for use in repair or reconstruction of damaged tissue as well as other surgical procedures, The device is particularly suited for repair of hernias and similar tissue damage requiring surgical placement and fixation of a patch-like material at the repair site.

The present invention is set out in appended claim 1.

The invention has two principle components. One component is an implantable device in a planar form. The other component is a resilient polymeric support member designed to assist in deployment and positioning of the implantable device at a surgical site. The resilient support member is adhered to the implantable device in such a way as to permit removal of the support member from the implantable device following placement of the implantable device in a recipient. The removal preferably occurs in a single step. The resilient support member is removed either manually or with surgical instruments. The resilient support member is also in planar form.

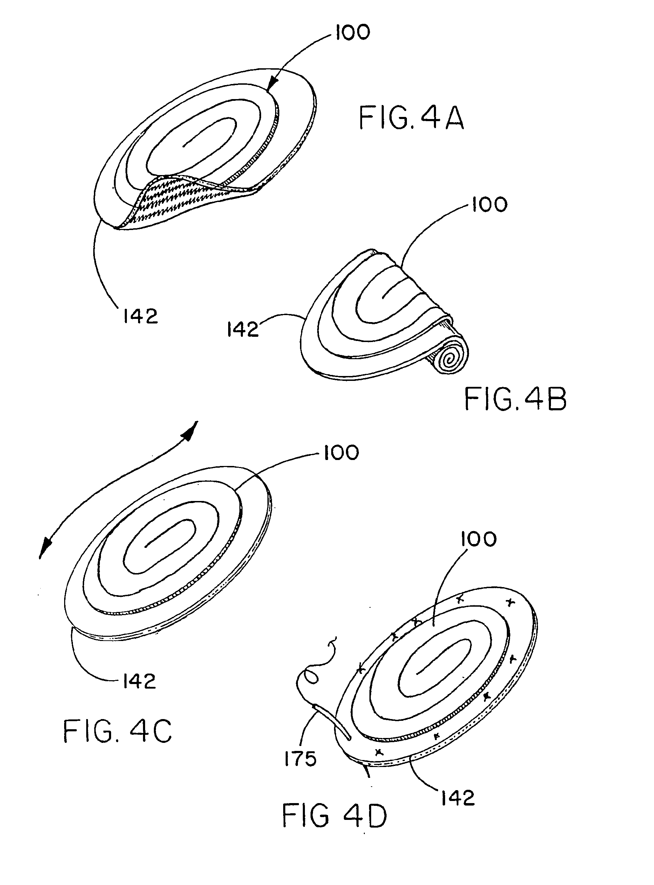

The combined planar materials are sufficiently pliable to permit the invention (Figure 4A) to be rolled, folded, or otherwise compacted in form (Figure 4B) and delivered with laproscopic instruments or other conventional surgical techniques. Following delivery of the invention to a surgical site, the resilient support member readily recovers from the compacted form to return to its original planar form. As the support member returns to its original planar form, the resilience of the support member causes the adhered implantable device to readily change from the compacted form to the original planar configuration. As the invention assumes a planar form at a surgical site (Figure 4C), the support member enables the implantable sheet material to be easily manipulated, positioned, and secured to tissues of the surgical site with surgical fasteners (Figure 4D).

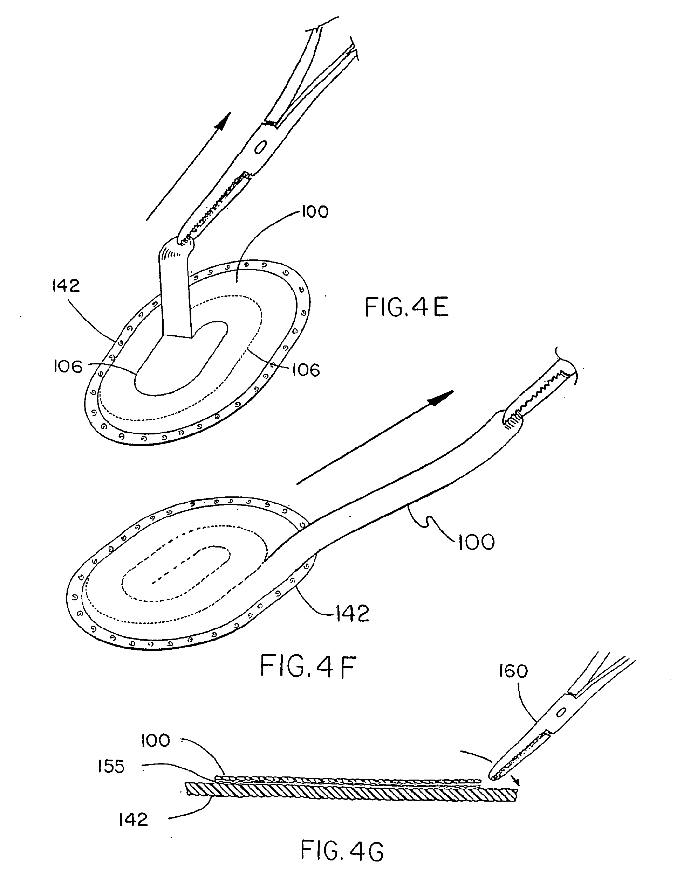

Once the implantable device is secured, a part of the support member is pulled upon to initiate release of the adhesive bonds holding the support member to the implantable device (Figure 4E). As the support member continues to be pulled, the remaining adhesive bonds progressively break until the support member is released from the implantable device (Figure 4F). The support member is then extracted from the surgical site.

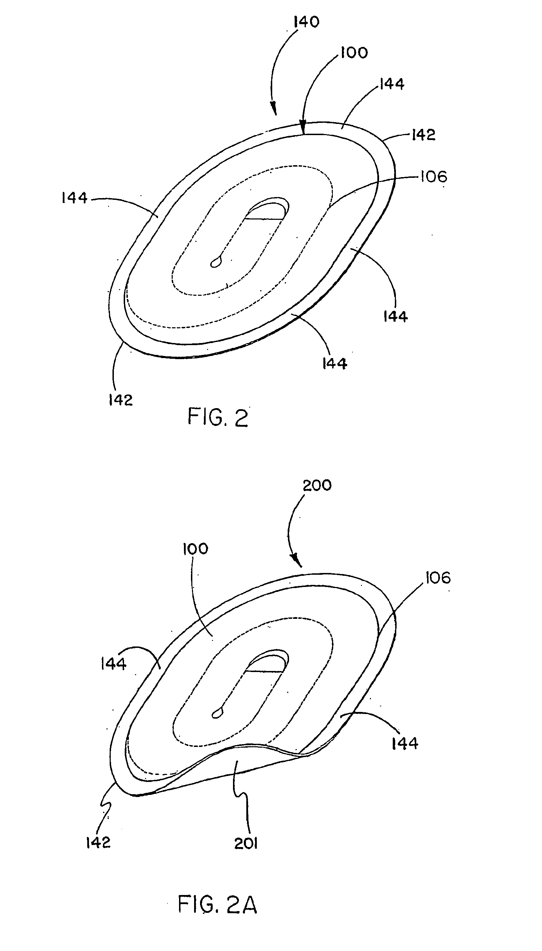

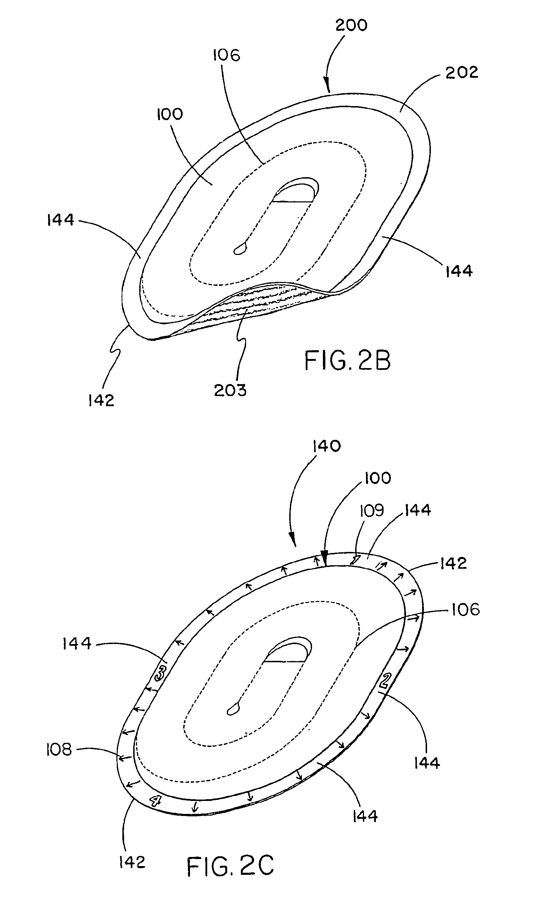

In some embodiments, the removable support member covers only part of the surface area of the implantable device (Figures 2, 2A, 2B, 5-7, *et al.*)*.* This leaves the border area of the implantable device exposed and available for fixation with sutures, staples, tacks, or other surgical fasteners. In these and other embodiments, the present invention can have letters, numbers, and other characters or features that aid in visual orientation of the invention with respect to a surgical repair site. A particularly preferred visual aid involves the use of different colors for the implantable device and the support member. In addition to the visual aids, the support member can be constructed to provide tactile distinctions between different sides of the invention as well as tactile distinctions between the implantable device and the support member.

One embodiment of the present invention is a medical device comprising a resilient support member and an implantable device attached to the resilient support member with an adhesive having a bond strength sufficient to hold the resilient support member and implantable device together during implantation procedures and allow the resilient support member to be removed from the implantable device once the implantable device is positioned within a body, wherein the resilient support member assists in deployment and placement of the implantable device during implantation procedures and is completely removed following implantation of the implantable device.

Another embodiment of the present invention is a medical device comprising an implantable sheet of flexible polymeric material having a surface area and a perimeter, and a resilient polymeric support member releasably adhered to at least a portion of a surface of said implantable sheet material, wherein said support member has a surface area less than said surface area of said implantable sheet and lies within said perimeter of said implantable sheet, and wherein said support member aids in deployment of said implantable sheet in a recipient and is removable from said implantable sheet following deployment of said implantable sheet in said recipient.

Each embodiment of the present invention can have an anti-microbial agent associated therewith.

Other features of the present invention will become apparent from the following detail description of the invention when taken in connection with the accompanying drawings. It is understood that the drawings are designed for the purpose of illustration only and are not intended as a definition of the limits of the invention.

### BRIEF DESCRIPTION OF THE DRAWINGS

Figure 1 illustrates a top plan view of a support member of the present invention.
Figure 1A illustrates a top plan view of a support member of the present invention.
Figure 1B illustrates an exploded cross-sectional view the present invention showing the relationship of the components as they are placed in a vacuum laminating fixture as shown in Figure 8.
Figure 2 illustrates a perspective view of the present invention.
Figure 2A illustrates a perspective view of the present invention with a corner of the implantable sheet material folded up to show a smooth texture of one surface of the sheet material.
Figure 2B illustrates a perspective view of the present invention with a corner of the implantable sheet material folded up to show a roughened texture of one surface of the sheet material.
Figure 2C illustrates a perspective view of the present invention with reference characters in the border area of the implantable device.
Figure 3A illustrates an exploded view of the present invention.
Figure 3B illustrates an exploded view of the present invention with a release agent applied between the support member and the implantable sheet material.
Figure 3C illustrates an exploded view of the present invention with an adhesive sheet placed between the support member and the implantable sheet material.
Figure 3D illustrates an exploded view of the present invention with a support member having substantially the same surface area as an implantable sheet material.
Figures 4A - 4D illustrate the present invention being compacted, unrolled, and afixed with surgical fasteners.
Figures 4E and 4F illustrate the support member of the present invention being removed from the implantable sheet material.
Figure 4G illustrates a cross-section of the present invention with a support member of sufficient thickness to provide a tactile step.
Figures 5 -7 illustrate different support member configurations releasably adhered to implantable materials of the present invention.
Figure 8 illustrates a cross-sectional view of a vacuum-laminating press useful in making the present invention.
Figure 9 illustrates two partial cross-sectional views of support members of the present invention having different framework structures associated with the support member.

### DETAILED DESCRIPTION OF THE INVENTION

The preferred medical device of the present invention is a composite of a resilient support member attached to an implantable sheet of flexible polymeric material suitable for use as a tissue repair material. The support member is attached to the implantable sheet material with an adhesive having a bond strength sufficient to hold the resilient support member and the implantable sheet material together during implantation procedures, while remaining sufficiently weak to allow the resilient support member to be separated from the implantable sheet material with conventional surgical techniques and removed from the implantable sheet material once the implantable sheet material is positioned within a recipient's body. Resilient support members attached to implantable devices with this adhesion scheme are referred to herein as being "releasably adhered" to the implantable devices. Both components of the preferred composite have a planar form.

As seen in Figure 2, for example, the preferred implantable device of the present invention (140) is a generally planar sheet of flexible, tissue-compliant, biocompatible polymeric material (142). Suitable polymeric materials include, but are not limited to, polypropylene, polyethylene, nylon, and polytetrafluoroethylene. The preferred polymeric material is an expanded, porous, polytetrafluoroethylene made according to U.S. Patent Nos. 3,953,566 and 4,187,390, both issued to Gore. There are two implantable sheet materials that are most preferred. One most preferred implantable sheet material is a tissue repair patch made of porous expanded polytetrafluoroethylene (ePTFE) available from W.L. Gore & Associates, Inc., Medical Products Division, Flagstaff, AZ under the tradename GORE-TEX^{®} DUALMESH^{®} Biomaterial as part number 1 DLMC04. The other most preferred implantable sheet material is a porous expanded polytetrafluoroethylene (ePTFE) material with an anti-microbial agent associated therewith. An antimicrobial treatment may be provided on the implantable sheet per, for example, U.S. Patent No. 5,019,096 issued to Fox, Jr., et al. The final product is available from W.L. Gore & Associates, Inc., Medical Products Division, Flagstaff, AZ under the tradename GORE-TEX^{®} DUALMESH^{®} PLUS Biomaterial as part number 1 DLMCP04. These most preferred materials have an oval shape with sizes in a range from 7.5 cm X 10.0 cm to 26.0 cm X 34.0 cm. Other planar shapes such as circles, squares, triangles, and custom-fitted shapes are also contemplated for use in the present invention. Regardless of the shape, suitable implantable sheet materials range in size from as small as 1.0 cm X 1.0 cm to as large as 50.0 cm X 50.0 cm, with 5.0 cm X 5.0 cm to 40.0 cm X 40.0 cm sized pieces being preferred, and pieces in a range from about 7.0 cm X 7.0 cm to about 20.0 cm X 20.0 cm being most preferred.

Suitable polymeric materials for the support member include, but are not limited to: silicones, including silicone elastomers such as polydimethyl siloxane; polyurethanes, including ester, ether, and carbonate based-urethanes; fluoroelastomers, including tetrafluoroethylene and polypropylene copolymers; terpolymers of vinylidine fluoride, hexafluoropropylene, and tetrafluoroethylene; perfluoroelastomers, including copolymers of tetrafluoroethylene and perfluoro (methyl vinyl) ether; polyethylene, including both linear and branched types and copolymers, such as ethylene-vinyl acetate; polypropylene; thermoplastic olefin elastomers, such as polypropylene/ethylene-propylene rubber (EPDM) vulcanates and ethylene-octene copolymers; styrenic resins, including polystyene and styrenic block coplymers, such as styrene-butadiene-styrene; polyamide (nylon); polyimide (*i.e*., Kapton^{®}); and polyester (*i.e*., Dacron^{®}). The preferred material is a silicone elastomer.

The shape of the resilient support member is preferably the same shape as the implantable sheet material. In preferred embodiments, the resilient support member has a surface area less than the surface area of the implantable sheet material and lies within the perimeter of the implantable sheet material. In the most preferred embodiment, shown in Figures 2 and 2B, this arrangement provides an unobstructed border area (144) on the implantable sheet material (142) that circumscribes the support member (100). The border area is available for sutures, staples, tacks, and/or other surgical fasteners. Useful border widths range from 0.3 cm to about 3.0 cm, with a preferred range from 0.5 cm to 2.0 cm, and a most preferred range between 0.8 cm to 1.2 cm.

With resilient support members having a thickness in a range from about 0.05 mm to about 2.0 mm, the thickness of the resilient support member can serve as an important feature of the present invention. During laproscopic procedures, for example, a clearly perceivable tactile change is felt when manually operated surgical instruments (160) are moved across the surface of the resilient support member and dropped off an edge of the support member onto the implantable sheet material (Figure 4G, arrow). A surgeon can take advantage of this "tactile step" to detect the border area of the implantable sheet material by feel. Knowing the location of the border area of the implantable sheet material permits the surgeon to confine placement of surgical fasteners to the border area and refrain from placing fasteners through the removable support member.

In addition to a tactile step, differences in surface characteristics between the implantable sheet material and the support member material can also provide additional tactile feedback to a surgeon. These tactile characteristics include, but are not limited to, surface texture, hardness, and/or lubriciousness.

The support member is adhered to the implantable sheet material with an adhesion scheme that permits the adhesive bonds to be broken with the type of manual forces used during conventional surgical techniques. Adhesion is a complex subject involving combined mechanical and physico-chemical phenomena operating simultaneously with any given adhesive or adhesion scheme. Accordingly, no single theory is adequate to explain adhesion.

Generally speaking, however, adhesives work by one of two mechanisms. The first mechanism is based on a thermodynamic model attributed to Sharpe and Schonhorn (L.H. Sharpe and H. Schonhorn, Chem. Eng. News 15:67 (1963)). The model is based on a belief that interatomic and intermolecular forces established at an interface between a substrate and an adhesive cause the adhesive to adhere to the substrate. The most common interfacial forces are thought to result from van der Waals and Lewis acid-base interactions. Adequate wetting of the substrate surface with an adhesive is another important aspect of forming adhesive bonds. With regard to the present invention, releasable adhesive bonds include formation of physico-chemical bonds between the support member, the implantable sheet material, and the adhesive (or within the adhesive itself) that are capable of holding the substrate materials together during implantation procedures. Following implantation, the adhesive bonds are broken and the adhered support member released from the implantable sheet material by applying sufficient mechanical force to the invention to physically destroy portions of the support member and/or portions of the implantable sheet material bonded by the adhesive or to disrupt the adhesive itself.

The other adhesion mechanism is a mechanical interlocking, or anchoring, of an adhesive material with cavities, pores, asperities, or other surface topographies of a substrate material (J.W. MacBain and D.G. Hopkins, J. Phys. Chem. 29:88 (1925)). Porous materials may also have subsurface openings that become filled and interlocked with an adhesive material. When a resilient support member of the present invention is releasably adhered to an implantable sheet material through mechanically interlocking adhesive bonds, the adhesive bonds can be broken, and the adhered support member released from the implantable sheet material, by applying sufficient mechanical force to the invention to break, burst, or otherwise disrupt the interlocking adhesive holding the two components together.

Though distinct in theory, both of these mechanisms are operable in forming most adhesive bonds, albeit to different degrees.

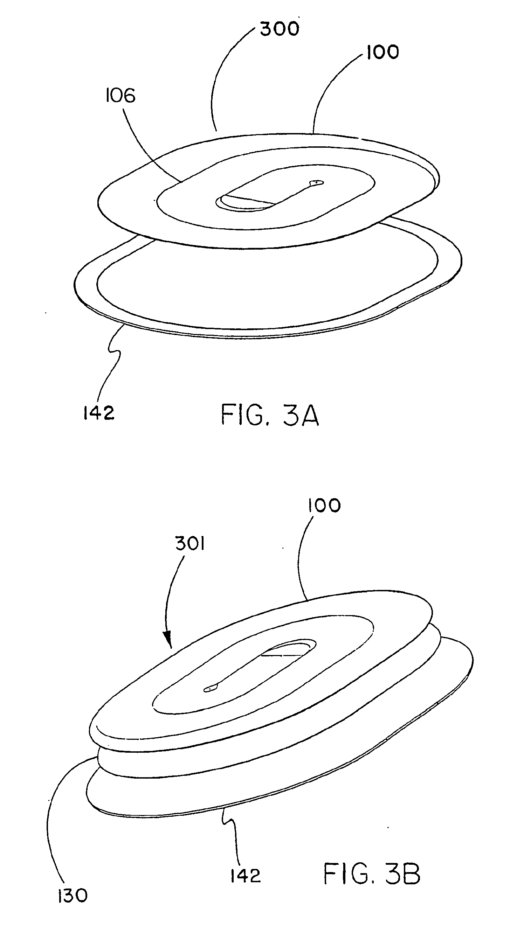

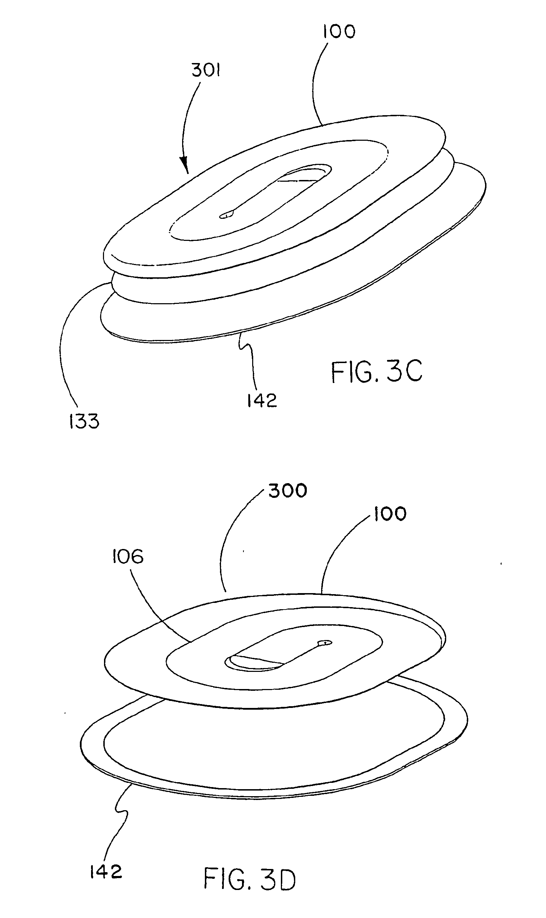

In some embodiments (*e.g*., Figure 3B), it may be desirable to employ an abhesive material (130) to aid in release of the resilient support member (100) from the implantable sheet material (142). The abhesive materials can be in the form of silicone release coatings, room-temperature-vulcanizing (RTV) molding products, or similar release systems.

The adhesion of the support member to the implantable sheet material may be accomplished by either softening the surface of the support member that interfaces with the device by the application of solvents or heat, or by the use of a suitable adhesive material.

For adhering the support member through the use of solvents or heat, it is preferable to use a support member constructed of a thermoplastic resin, including those listed above. The interfacing surface of the support member may then be softened by a suitable solvent, such as acetone for polyurethanes, or by applying heat. When applied in this condition the softened surface of the support member would then wet and conform to the implantable sheet substrate to achieve a suitable bond. The support member my also be adhered by overmolding or induction heating (*i.e*., with metallic reinforcements).

Suitable adhesive materials for use in the present invention include, but are not limited to, silicone (polydimethyl siloxane-based) elastomers, including low and high consistency types of various cure chemistries, such as condensation, addition, and peroxide; silicone gels; polyurethanes, including liquid, hot-melt/B-staged, and gum types; acrylates, including cyanoacrylate (moisture cure), ultraviolate curable resins, such as methyl methacrylate and methyl methacrylate modified resins; and a variety of resins that may be used for pressure sensitive adhesives (PSA) and hot melt adhesives, including styrenic block copolymers and terpolymers (i.e., styrene-isoprene-styrene), ethylene-propylene-diene copolymers and terpolymers, ethylene vinyl acetate, acrylics, urethanes, silicones (PSA only).

Methods of applying adhesive materials to either the resilient support member component or implantable device component include, but are not limited to, dispensing the adhesive manually, mechanically, or automatically in a pattern or over the entire surface area of the component. The adhesive can be applied as a thin film, coated, sprayed, and/or printed. An appropriate hot-melt or PSA adhesive can also be applied by the above methods with adequate heat, and may also include techniques such as induction heating (*i.e*., with metallic reinforcements), and microwaving (for support members and/or adhesives that possess some polarity). Regardless of the technique, the adhesive is preferably allowed to set on either component for a period of time to allow the adhesive to begin to solidify or cure. The preferred dwell time for this step ranges from about fifteen (15) seconds to about five (5) minutes.

As seen in Figure 3C, adhesive sheets with adhesive material on both sides of the sheets (133) can also be used between the support member component (100) and the implantable sheet material component (142) to releasably adhere the components together. Preferred adhesives for these materials are pressure-sensitive adhesives.

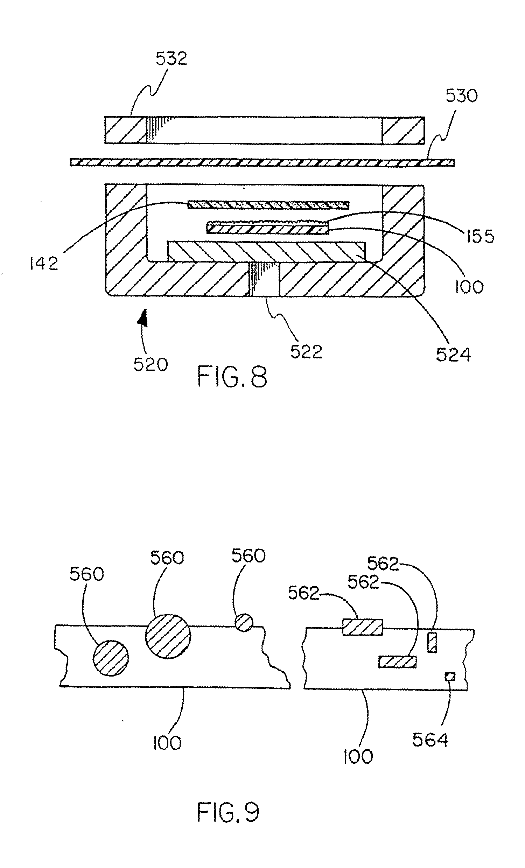

Once an appropriate adhesive material is applied to one or both components of the present invention, mechanical pressure is applied to the components to releasably adhere the components together. A preferred method includes a vacuum fixture as shown in Figure 8 and discussed in Example 1, below. Additional methods include, but are not limited to, placing a weighted flattened plate on the components and applying mechanical force to the components with a roller or similar device.

The support member component (100) of the present invention (Figure 2, *et seq*.) is made of a resilient polymeric material in generally planar form. The support member can be constructed of a single material or plurality of materials in the form of a composite. The resilience of the support member can be an inherent property of the polymeric material or supplied to the polymeric material with a framework structure. Shown in Figure 9 are partial cross sectional views of support members 100 incorporating various framework structures. Shown are circular shaped framework structures 560 embedded within the support member 100. Also shown are rectangular framework structures 562 and a square framework structure 564, all embedded within the support member 100, support structures 560, 562, 564 can have any cross section shape in order to supply resilience to the support member. For example, a support member can have an essential, circular, oval, triangle, square, rectangle or other polygon cross section. The support structure can also be formed from composites, laminates, weaves, yarns or other suitable forms.

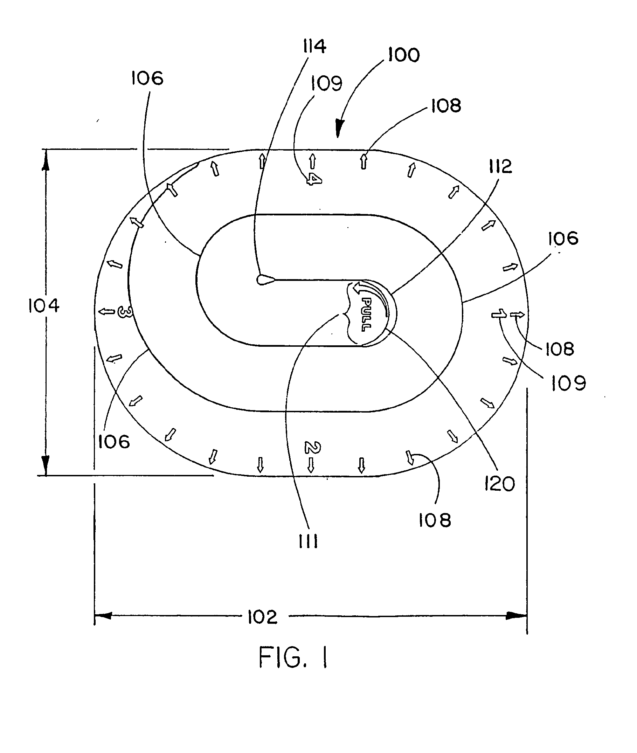

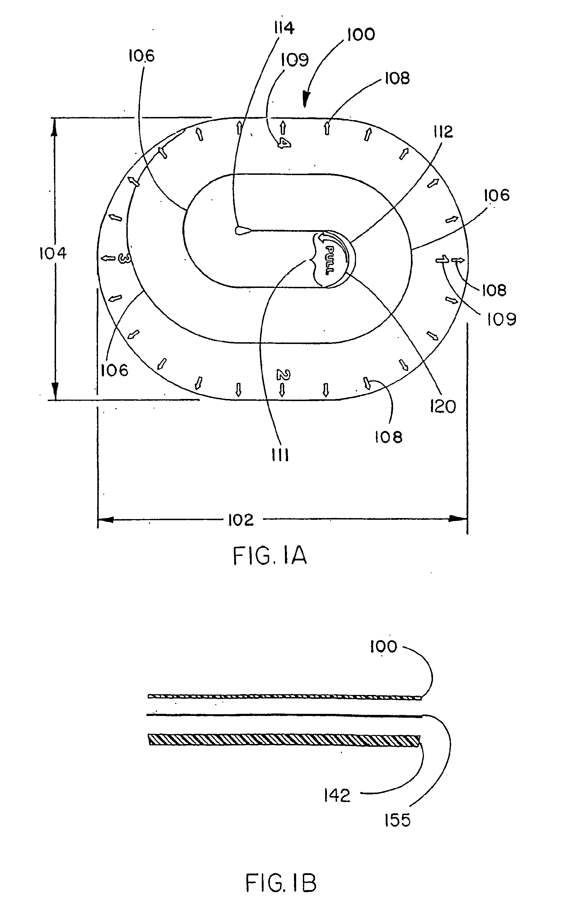

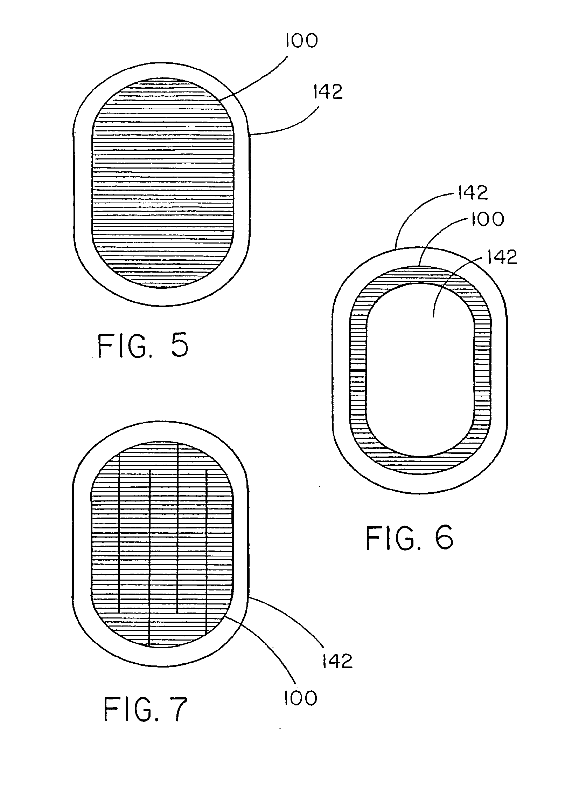

While various forms are contemplated for the removable resilient support member (Figures 5-7), the preferred form is a coil (Figure 1) having a free end (110) at the center of the coil. The free end is a portion of the support member that is not adhered to the implantable sheet material. The free end can protrude above the surface of the support member or lie in the same plane as the support member and be lifted from the surface of the implantable sheet material manually or with surgical instruments. Access to the free end (110) can be provided by a hole (112) cut in the support member (100) immediately adjacent the free end (110). Lifting and pulling the free end (Figure 4E) begins the removal of the support member from the implantable sheet material. As the free end is pulled, the adhesive bonds are broken and the support member released from the implantable sheet material. As the support member is released, it uncoils from the implantable sheet material in a continuous length until it is completely removed from the implantable sheet material (Figure 4F). Pulling the support member from the central areas of the implantable sheet material distributes the pulling forces more evenly across the sheet material. This, in turn, reduces the chance the pulling forces will concentrate in a particular spot on the implantable sheet material and damage or tear a surgical fastener holding the implantable sheet material in place.

In other embodiments, the removable support member has a freeable end (120, Figure 1A). The freeable end is a portion of the releasably adhered support member that can be grabbed and pulled away from the implantable sheet material to release the adhesive bond and initiate removal of the support member from the implantable sheet material. In some embodiments, access to the freeable end (120) can be provided by a hole (112) cut in the support member (100) immediately adjacent the freeable end (120). Lifting and pulling the freeable end releases the adhesive bond holding the freeable end to the implantable sheet material. As the freeable end is pulled further, it becomes completely detached from the implantable sheet material to form a free end (Figure 4E). Continuing to pull the free end causes the removable support member to completely detach from the implantable sheet material as previously described (Figure 4F).

Having a free end, or freeable end, located in a central area of the support member makes access to the end easy and not dependent on a particular orientation of the invention at a surgical site.

The coil, or other support member form, is preferably delimited by a narrow strip of mechanically weakened material that preferentially breaks away, or tears, from adjacent support member material when the support member is removed. Such a strip of mechanically weakened support member material is referred to herein as a "tear line" (106, Figure 1, *et seq.*). The tear line can be in the form of a continuous cut of determined width and depth, a series of perforations that combine to form an outline, or a similar mechanical weakening of the removable support member material. If perforations are used, it is preferred to space the perforations closely together in the outline. When a free end is pulled upon, the close spacing of the perforations causes the support member material immediately between the perforations to tear, break away, and produce a tear line. The preferred support member has a tear line in the from of a continuous cut having a depth of cut, expressed as a percent of the support member thickness, in a range from 20% to 100%, with a preferred depth of cut in a range from 50% to 90%, and a most preferred depth of cut in a range from 70% to 80%.

As the support member of the preferred embodiment is removed along its tear lines, the support member assumes an elongate configuration of set width. Having the elongating support member maintain a set width during removal permits the support member to be easily and reliably passed through laproscopic and similar small bore surgical instruments or surgical openings.

An implantable device is described. The implantation aid comprises a resilient member, an adhesive attached to the resilient member, the adhesive having sufficient bond strength to attach the resilient member to an implantable device during implantation procedures, while allowing the resilient member to be readily removed from the implantable device once the implantable device is positioned within a body, wherein the resilient member assists in deploying and positioning the implantable device during implantation procedures while being completely removed following implantation of the implantable device. The implantation aid has a length and a width, with the length exceeding the width. The width is at least one centimeter.

### EXAMPLES

### EXAMPLE 1

This example describes the construction of a preferred embodiment of the present invention. Following formation of a support member, adhesive is applied to the support member and the combination pressed together with an implantable sheet material.

As shown in Figure 1, the support member (100) had a length (102) of about 17 cm (∼ 6.7"), a width (104) of about 13cm (- 5"). For a support member made of an 80A durometer silicone compound, the preferred thickness is about 0.3 mm to about 0.5 mm. In this example, the thickness of the support member was about 0.4mm (- 0.16").

The support member (100) had a tear line (106) in the form of a continuous cut delimiting a coil form. The tear line (106) was molded into the support member and had a maximum width of about 0.3mm (- 0.012") and a depth of about 0.25mm (- 0.01"). The tear line, therefore, did not penetrate or extend through the full thickness of the support member. By limiting the depth of the tear line, the support member could be processed in the form of a sheet and not "uncoil" during subsequent handling.

The support member had markings (108) pad-printed onto the top surface. These markings (108) were adapted to aid visual orientation of the present invention with respect to an implantation site. In this embodiment, the markings (108) included a series of arrows, spaced about 2cm (- 0.8") apart, located about the periphery of the support member. These arrows serve as visual staple references or as peripheral length references during deployment and implantation. Other markings (109) included numeric references (shown as 1, 2, 3, and 4) located at 90 degree increments along the support member periphery. These numeric references serve as general visual orientation marks similar to north, south, east and west on a compass. Other markings included instructions (111) in the form of a curved arrow and the word "PULL" located on free end (110) of the coiled support member. This free end (110) serves as a "pull-tab," or tear-start, allowing a surgeon to grasp and pull the free end (110) to initiate the removal of the coiled support member. A crescent shaped through-hole (112) was molded into the support member to aid in access to free end (110).

A tear-relief through-hole (114) was molded into the support member to prevent undesirable tearing of the support member across the first, 180 degree, turn of the coil pattern. By tearing only along tear line (106), tear-relief (114) allows the support member to be uncoiled and removed as a single linear strip.

The resilient support member was fabricated from a medical grade silicone elastomer, part number Q7-4780 available from Dow Corning, Midland, MI. The resilient support member was tinted green using about 1.4 parts per hundred rubber (phr) Silcopas GREEN 176, available from Gayson Specialty Dispersions, Barberton, OH. The green tint rendered a visual contrast to the exposed border of the white or light brown implantable sheet material. A matt finish was provided to the top and surfaces of the resilient support member to prevent the support member from sticking or adhering to itself when rolled for insertion into a trocar. The matt finish aids in releasably adhering the support member. The matt finish was created by a dry blast, glass bead, finish (D1 SP1) applied to surfaces of a mold used to fabricate the resilient support member. The markings/labels were pad printed onto the resilient support member using medical grade silicone ink part number SS70117, available from VESTA, Inc., Franklin, WI.

Once the resilient support member was fabricated, an adhesive was applied to the bottom, unmarked side, of the support member with an automatic dispensing system. The system was a MillRight™ series 18 programmable X/Y table available from MHO Corporation, Emeryville, CA. The programmable table was outfitted with a pneumatic dispensing system from EFD (E. Providence RI). The dispensing system consisted of an adjustable high pressure valve (model 736), a 1.5 mm (0.06 inch) ID metal nozzle (part number 5014-1/4 NPT) and a controller (model Valvemate 7000)). The adhesive was applied by first aligning the resilient support member onto an X/Y table. A serpentine pattern of MED-1137 adhesive (NuSil Technology, Carpinteria, CA) was then dispensed onto the support member at 50 inches per minute (ipm). The head pressure on the valve was ∼ 0,69 MPa (100 psi) and the tip clearance from the support member was 1 mm (∼ 0.04 inches). The serpentine pattern had an approximate line to line center spacing of about 4 mm (∼ 0.16"). The resilient support member had a surface area of about 180 cm² (∼ 28 in²) and about 5 grams of adhesive was applied thereto. The adhesive was left on the support member for about fifteen (15) seconds before proceeding to the next step.

The adhesive-coated support member was then bonded to an implantable sheet. Shown in Figure 8 is a cross-sectional view of a vacuum laminating fixture. Figure 8 illustrates a laminating fixture (520), having a vacuum port (522) and a porous metal plate (524). The support member (100) with the applied adhesive (155) was placed onto the porous plate (524). An implantable sheet (142) was then aligned onto the exposed adhesive (155). The support member (100) and implantable sheet (142) were oriented by the use of alignment marks on the porous plate (524). A silicone sheet (530) was placed onto the fixture (520) and clamped to the fixture by a ring clamp (532). A vacuum was applied to the vacuum port, causing the silicone sheet (530) to deflect and apply a compressive load to the implantable sheet (142), adhesive (155) and support member (100). The vacuum was applied for about 10 seconds, causing the dispensed pattern of adhesive (155), to "flatten" under the compressive load. The implantable sheet (142) was an expanded polytetrafluoroethylene (ePTFE), 15cm X 19cm oval hernia repair patch, tradenamed GORE-TEX^{®} DUALMESH^{®} Biomaterial available from the Medical Products Division of W.L. Gore & Associates, Inc. (Flagstaff, AZ) as part number 1 DLMC04. As seen in Figure 2B, the GORE-TEX^{®} DUALMESH^{®} Biomaterial product (142) has a different texture (203) on each side of the sheet. One side (202) is designed to prevent or limit tissue adhesions or other tissue attachments thereto. The other side (203) is roughened to encourage tissue attachment or ingrowth of cells or cellular process therewithin. The GORE-TEX^{®} DUALMESH^{®} Biomaterial was oriented so that the "tissue adhesion barrier" side (202) was against the adhesive (155). The silicone sheet (530) was about 0.4mm (∼ 0.016") thick. The GORE-TEX^{®} DUALMESH^{®} Biomaterial (142), adhesive (155), and support member (100) components were then removed from fixture (520).

The combined components were placed in an environmental chamber at about 25°C and 50% relative humidity for about 48 hours to cure the adhesive. During the curing process, the chamber was purged with fresh air at a change-over rate of approximately one per minute to remove any vaporous solvents or by-products (e.g., acetic acid) from the adhesive cure reaction. The bond strength of the adhesive was such that the support member was releasably adhered to the implantable sheet material. As seen in Figure 2, the medical device (140) had an exposed border area (144) of the implantable sheet (142) that completely circumscribed the support member (100).

### Example 2

This example describes the construction of a resilient polymeric support member releasably adhered to an implantable sheet material having an anti-microbial treatment applied thereto.

A resilient support member was constructed according to Example 1. An implantable ePTFE sheet material with an anti-microbial treatment was obtained from the Medical Products Division of W.L. Gore & Associates, Inc., Flagstaff, AZ under the tradename GORE-TEX^{®} DUALMESH^{®} PLUS Biomaterial as part number 1DLMCP04.

The support member was releasably adhered to the implantable sheet material according to Example 1 with the exception that the dwell time between adhesive application and component assembly was increased to about two (2) minutes from the fifteen (15) second dwell time described in Example 1. The increased dwell time was necessitated by the anti-microbial treatment applied to the implantable ePTFE sheet material.

### Example 3

This example describes the construction of an embodiment of the present invention using a medical grade polyurethane material available from Dow Chemical, Midland, MI under the tradename Pellethane® Thermoplastic Polyurethane Elastomers (part number 2363-80) for the support member. The support member is molded from the polyurethane material to the dimensions described in Example 1. Following formation of the support member, adhesive is applied to the support member as described in Example 1 and the combination pressed together with an implantable sheet material as described in Example 1. This resulted in the urethane-based support member releasably adhered to the implantable sheet material.

### Example 4

This example describes the construction of an embodiment of the present invention. Following formation of a support member, adhesive is applied to the support member and the combination pressed together with a composite implantable sheet material.

The support member is made according to either Example 1 or Example 8 with dimensions that provide an exposed border area on the composite implantable sheet of about 1.0cm.

The composite implantable sheet is an 20.3cm (8 inch) by 25.4cm (10 inch) hernia repair patch made of a layer of a monofilament knitted polypropylene and a layer of expanded polytetrafluoroethylene (ePTFE). The composite implantable sheet is taught in U.S. Patent No. 5,593,441, issued to Lichtenstein, et al., and available under the tradename BARD^{®} COMPOSIX™ Mesh from Davol, Inc., a subsidiary of C.R. Bard Inc., Cranston, RI., as part number 0113810. The BARD^{®} COMPOSIX™ Mesh material is oriented so that its ePTFE side is against the adhesive.

The adhesive material is applied to the support member as described in Example 1. The component parts are releasably adhered as also described in Example 1.

### Example 5

This example describes the construction of an embodiment of the present invention using a two-part adhesive material to releasably adhere the support member to the implantable sheet material.

A two-part adhesive material is obtained from Hardman, Inc., a division of Harcros Chemicals Inc., Belleville, NJ, having a shore hardness of 85A under the part number 4024. Following formation of a support member as described in Example 1, the two parts of the adhesive are mixed together at room temperature and formed into a thin film (*i.e*., less than 0,13 mm (0.005") on the support member. The adhesive is allowed to gel for about five (5) minutes until tacky to the touch. An implantable sheet material is adhered to the support member as described in Example 1. The result is a support member releasably adhered to the implantable sheet material.

### Example 6

This example describes the construction of an embodiment of the present invention using a thin polyethylene sheet material with a pressure-sensitive acrylic adhesive on both sides of the material to releasably adhere a support member to an implantable sheet material.

A support member is constructed according to Example 1. An implantable sheet material is obtained as described in Examples 1 or 4, for example. An adhesive sheet is obtained from Avery Dennison Specialty Tape Division under the tradename Avery Dennison™ MED 3044 as part number 57809. The sheet consists of a 76 µm (3 mil) thick clear polyethylene film coated on both sides with a non-sensitizing acrylic pressure-sensitive adhesive.

The adhesive sheet is cut to fit the support member and pressed into place on the support member. The implantable sheet material is then pressed into place on the opposite side of the pressure-sensitive adhesive sheet to releasably adhere the support member to the implantable sheet material.

### Example 7

This example describes the construction of a preferred embodiment of the present invention. Following formation of a support member with a freeable end, adhesive is applied to the support member and the combination pressed together with an implantable sheet material.

The support member in this embodiment is made according to Example 1 with the exception that adhesive material was applied to the support member so as to provide a freeable end (120, Figure 1A).

The remaining materials and methods are the same as in Example 1.

### Example 8

This example describes the construction of a support member with an adhesive applied to one side thereof.

As shown in Figure 1, the support member (100) has a length (102) of about 17 cm (∼ 6.7"), a width (104) of about 13cm (- 5"). For a support member made of an 80A durometer silicone compound, the preferred thickness is about 0.3 mm to 0.5 mm. In this example, the thickness of the support member is about 0.4mm (- 0.16").

The support member (100) has a tear line (106) in the form of a continuous cut delimiting a coil form. The tear line (106) is molded into the support member and has a maximum width of about 0.3mm (∼ 0.012") and a depth of about 0.25mm (∼ 0.01 "). The tear line, therefore, did not penetrate or extend through the full thickness of the support member. By limiting the depth of the tear line, the support member could be processed in the form of a sheet and not "uncoil" during subsequent handling.

The support member has markings (108) pad-printed onto the top surface. These markings (108) are adapted to aid visual orientation of the present invention with respect to an implantation site. In this embodiment, the markings (108) include a series of arrows, spaced about 2cm (- 0.8") apart, located about the periphery of the support member. These arrows serve as visual staple references or as peripheral length references during deployment and implantation. Other markings (109) include numeric references (shown as 1,2 3 and 4) located at 90 degree increments along the support member periphery. These numeric references serve as general visual orientation marks similar to north, south, east and west on a compass. Other markings include instructions (111) in the form of a curved arrow and the word "PULL" located on free end (110), or freeable end (120), of the coiled support member. This free end (110), or freeable end (120), serves as a "pull-tab," or tear-start, allowing a surgeon to grasp and pull the free end (110) to initiate the removal of the coiled support member. A crescent shaped through-hole (112) is molded into the support member to aid in formation of free end (110), or freeable end (120).

A tear-relief through-hole (114) is molded into the support member to prevent undesirable tearing of the support member across the first, 180 degree, turn of the coil pattern. By tearing only along tear line (106), tear-relief (114) allows the support member to be uncoiled and removed as a single linear strip.

The resilient support member is fabricated from a medical grade silicone elastomer, part number Q7-4780 available from Dow Corning, Midland, MI. The resilient support member is tinted green using about 1.4 parts per hundred rubber (phr) Silcopas GREEN 176, available from Gayson Specialty Dispersions, Barberton, OH. The green tint renders a visual contrast to the exposed border of the white or light brown implantable sheet material. A matt finish is provided to the top and bottom surfaces of the resilient support member prevent the support member from sticking or adhering to itself when rolled for insertion into a trocar. The matt finish also aids in releasably adhering the support member. The matt finish is created by a dry blast, glass bead, finish (D1 SP1) applied to surfaces of a mold used to fabricate the resilient support member. The markings/labels are pad printed onto the resilient support member using medical grade silicone ink part number SS70117, available from VESTA, Inc., Franklin, WI.

Once the resilient support member is fabricated, an adhesive is applied to the bottom, unmarked side, of the support member with an automatic dispensing system. The system is a MillRight™ series 18 programmable X/Y table available from MHO Corporation, Emeryville, CA. The programmable table is outfitted with a pneumatic dispensing system from EFD (E. Providence, RI). The dispensing system consists of an adjustable high pressure valve (model 736), a 1,5 mm (0.06 inch) ID metal nozzle (part number 5014-1/4 NPT) and a controller (model Valvemate 7000). The adhesive is applied by first aligning the resilient support member onto an X/Y table. A serpentine pattern of MED-1137 adhesive (NuSil Technology, Carpinteria, CA) is then dispensed onto the support member at 0,02 m/s (50 inches per minute (ipm)). The head pressure on the valve is 0,69 MPa ∼ (100 psi) and the tip clearance from the support member is 1 mm (∼ 0.04 inches). The serpentine pattern has an approximate line to line center spacing of about 4 mm (∼ 0.16"). The resilient support member has a surface area of about 180 cm² (∼ 28 in²) and about 5 grams of adhesive is applied thereto.

## Claims

1. A medical device comprising:
an implantable sheet (142) of flexible polymeric material having a surface area and a perimeter; **characterized in that**
a resilient polymeric support member (100) is releasably adhered to at least a portion of a surface of said implantable sheet material, wherein said support member has a surface area less than said surface area of said implantable sheet and lies within said perimeter of said implantable sheet, wherein said support member has a sequence of perforations (106) that operate to form tear lines as said support member is removed from said implantable sheet, and wherein said support member aids in deployment of said implantable sheet in a recipient and is removable from said implantable sheet following deployment of said implantable sheet in said recipient.

2. The medical device of claim 1 wherein said support member lies completely within said border area of said implantable sheet material.

3. The medical device of claim 1 wherein said implantable sheet has a border area that circumscribes said support member.

4. The medical device of claim 1 wherein said implantable sheet further comprises an antimicrobial agent.

5. The medical device of claim 1 wherein said device further comprises an abhesive.

6. The medical device of claim 1 wherein said implantable sheet has one or more markings adapted to aid visual orientation of said medical device with respect to an implantation site.

7. The medical device of claim 6 wherein said markings are located in a border area of said implantable sheet.

8. The medical device of claim 1 wherein said support member has one or more markings adapted to aid visual orientation of said medical device with respect to an implantation site.

9. The medical device of claim 1 wherein said support member has a free end.

10. The medical device of claim 1 wherein said support member has a freeable end.

11. The medical device of claim 1 wherein said implantable sheet comprises a porous expanded polytetrafluoroethylene material.

12. The medical device of claim 11 wherein said porous expanded polytetrafluoroethylene material comprises a first layer and a second layer.

13. The medical device of claim 12 wherein said first layer is sufficiently porous to permit ingrowth of cells or cellular processes therewithin and wherein said second layer does not support attachment of tissue thereto.

14. The medical device of claim 1 wherein said polymeric support member comprises a silicone compound.

## Patentansprüche

1. Medizinvorrichtung, die aufweist:
eine implantierbare Folie (142) aus flexiblem Polymermaterial mit einer Oberfläche und einem Umfang; **dadurch gekennzeichnet, daß**
ein elastisches Polymerträgerelement (100) lösbar zumindest an einem Teil eine Oberfläche des implantierbaren Folienmaterials angeklebt ist, wobei das Trägerelement eine Oberfläche aufweist, die kleiner ist als die Oberfläche der implantierbaren Folie, und innerhalb des Umfangs der implantierbaren Folie liegt, wobei das Trägerelement eine Folge von Perforationen (106) aufweist, die beim Abziehen des Trägerelements von der implantierbaren Folie zum Bilden von Abreißlinien dienen, und wobei das Trägerelement das Einsetzen der implantierbaren Folie in einen Empfänger unterstützt und anschließend an das Einsetzen der implantierbaren Folie in den Empfänger von der implantierbaren Folie abziehbar ist.

2. Medizinvorrichtung nach Anspruch 1, wobei das Trägerelement vollständig innerhalb der Randfläche des implantierbaren Folienmaterials liegt.

3. Medizinvorrichtung nach Anspruch 1, wobei die implantierbare Folie eine Randfläche aufweist, die das Trägerelement begrenzt.

4. Medizinvorrichtung nach Anspruch 1, wobei die implantierbare Folie ferner ein antimikrobielles Mittel aufweist.

5. Medizinvorrichtung nach Anspruch 1, wobei die Vorrichtung ferner ein Abhäsivmittel aufweist.

6. Medizinvorrichtung nach Anspruch 1, wobei die implantierbare Folie eine oder mehrere Markierungen aufweist, die so angepaßt sind, daß sie die visuelle Ausrichtung des Medizinvorrichtung bezüglich einer Implantationsstelle unterstützen.

7. Medizinvorrichtung nach Anspruch 6, wobei sich die Markierungen in einer Randfläche der implantierbaren Folie befinden.

8. Medizinvorrichtung nach Anspruch 1, wobei das Trägerelement eine oder mehrere Markierungen aufweist, die so angepaßt sind, daß sie die visuelle Ausrichtung der Medizinvorrichtung bezüglich einer Implantationsstelle unterstützen.

9. Medizinvorrichtung nach Anspruch 1, wobei das Trägerelement ein freies Ende aufweist.

10. Medizinvorrichtung nach Anspruch 1, wobei das Trägerelement ein freimachbares Ende aufweist.

11. Medizinvorrichtung nach Anspruch 1, wobei die implantierbare Folie einen porösen Polytetrafluorethylen-Schaumstoff aufweist.

12. Medizinvorrichtung nach Anspruch 11, wobei der poröse Polytetrafluorethylen-Schaumstoff eine erste Schicht und eine zweite Schicht aufweist.

13. Medizinvorrichtung nach Anspruch 12, wobei die erste Schicht ausreichend porös ist, um das Einwachsen von Zellen oder zelluläre Prozesse in der ersten Schicht zuzulassen, und wobei die zweite Schicht eine Bindung von Gewebe daran nicht unterstützt.

14. Medizinvorrichtung nach Anspruch 1, wobei das polymere Trägerelement eine Siliconverbindung aufweist.

## Revendications

1. Dispositif médical comprenant:
une feuille implantable (142) d'un matériau polymère flexible possédant une aire de surface et un périmètre; **caractérisé en ce que**:
un élément de support polymère élastique (100) est adhéré de manière amovible à au moins une portion d'une surface dudit matériau de la feuille implantable, dans lequel ledit élément de support possède une aire de surface inférieure à ladite aire de surface de ladite feuille implantable et se trouve à l'intérieur dudit périmètre de ladite feuille implantable, dans lequel ledit élément de support présente une séquence de perforations (106) qui fonctionne pour former des lignes de déchirure lorsque ledit élément de support est retiré de ladite feuille implantable et dans lequel ledit élément de support aide au déploiement de ladite feuille implantable dans un receveur et celui-ci peut être retiré de ladite feuille implantable à la suite du déploiement de ladite feuille implantable dans ledit receveur.

2. Dispositif médical selon la revendication 1, dans lequel ledit élément de support se trouve complètement à l'intérieur de ladite région de bordure dudit matériau de la feuille implantable.

3. Dispositif médical selon la revendication 1, dans lequel ladite feuille implantable possède une région de bordure qui limite ledit élément de support.

4. Dispositif médical selon la revendication 1, dans lequel ladite feuille implantable comprend en outre un agent antimicrobien.

5. Dispositif médical selon la revendication 1, où ledit dispositif comprend en outre un adhésif.

6. Dispositif médical selon la revendication 1, dans lequel ladite feuille implantable possède un ou plusieurs marquages adaptés pour aider à l'orientation visuelle dudit dispositif médical par rapport à un site d'implantation.

7. Dispositif médical selon la revendication 6, dans lequel lesdits marquages sont localisés dans une région de bordure de ladite feuille implantable.

8. Dispositif médical selon la revendication 1, dans lequel ledit élément de support possède un ou plusieurs marquages adaptés pour aider à l'orientation visuelle dudit dispositif médical par rapport à un site d'implantation.

9. Dispositif médical selon la revendication 1, dans lequel ledit élément de support possède une extrémité libre.

10. Dispositif médical selon la revendication 1, dans lequel ledit élément de support possède une extrémité libérable.

11. Dispositif médical selon la revendication 1, dans lequel ladite feuille implantable comprend un matériau de polytétrafluoroéthylène expansé poreux.

12. Dispositif médical selon la revendication 11, dans lequel ledit matériau de polytétrafluoroéthylène expansé poreux comprend une première couche et une deuxième couche.

13. Dispositif médical selon la revendication 12, dans lequel ladite première couche est suffisamment poreuse pour permettre la pénétration de cellules ou de processus cellulaires à l'intérieur de celle-ci et dans lequel ladite deuxième couche ne supporte pas la fixation d'un tissu sur celle-ci.

14. Dispositif médical selon la revendication 1, dans lequel ledit élément de support polymère comprend un composé de silicone.
